# EUROPEAN PATENT SPECIFICATION

(11) **EP 1 988 934 B1**
(45) Date of publication and mention of the grant of the patent: **20.10.2010**
(21) Application number: 07785096.4
(22) Date of filing: 16.08.2007
(51) Int. Cl.: A61L 9/12

(54) **VOLATILE SUBSTANCE DISPENSING METHOD**
METHODE ZUR FREISETZUNG FLÜCHTIGER SUBSTANZEN
METHODE DE DISTRIBUTION DE SUBSTANCE VOLATILE

(30) Priority: 24.08.2006 EP 06291363
(43) Date of publication of application: 12.11.2008
(73) Proprietor: Givaudan SA, 1214 Vernier (CH)
(72) Inventor: BLONDEAU, Philippe, F-75019 Paris (FR); BRESSON BOIL, Alice, F-95220 Herblay (FR)
(74) Representative: Givaudan Patents
(86) International application number: PCT/CH2007/000401
(87) International publication number: WO 2008/022477

(56) References cited:
- WO-A-94/06480
- WO-A-03/086490
- WO-A-2006/128316
- FR-A- 2 459 766
- US-A- 4 889 286

## Description

This invention relates to a method for dispensing volatile substances, and more particularly to a method of using a membrane-based dispensing device for the delivery of volatile substances from a liquid to an ambient environment by evaporation as defined in claim 1.

Membrane-based dispensing devices for the dispensing into an ambient environment of volatile liquids such as fragrances, bactericides, fungicides and disinfectants are well known in the art. One very common type of such dispensing devices consists essentially of a reservoir containing the volatile liquid and a membrane covering the reservoir and contacting the volatile liquid. Such dispensing devices employ diffusion phenomenon to provide the motive dispensing force. The liquid phase evaporates through the membrane to the ambient environment. Such a device may additionally comprise auxiliary dispensing means, such as heating elements and/or fans.

While such devices are undoubtedly successful and have been commercially successful, they have certain practical drawbacks. One is a phenomenon called "habituation", that is, with continuous emission, people simply get used to the odour and cease to notice it. Much of the fragrance is therefore wasted to a certain extent. This can be overcome, but means of doing this have generally been both relatively complex and relatively expensive. For example, it is possible to provide programmable devices with automatically opening orifices and the like. Such expense and complexity is often not justified.

International Patent Application WO 94/06480 describes an emanator for volatile liquids comprising a laminated porous element.

In International Patent Application WO 2006/128316, published in 7 December 2006, there is disclosed a simple device that overcomes many of these disadvantages. This device uses a relatively thick membrane (from 0.1-5mm thick, preferably from 0.5-5mm), which is wetted with the volatile liquid and which then evaporates into the atmosphere when the liquid and the membrane are separated. While this works well, it suffers from the problem that, when the liquid in the membrane has fully evaporated, there is substantially no further emission of liquid, because the thick membrane does not readily permit the emission of evaporated liquid within the reservoir, necessitating the recharging of the membrane.

It has now been found that it is possible to overcome this problem and obtain both continuous emission of liquid and increased emission when required. The invention therefore provides a method of providing in an atmosphere both a continual supply of volatile liquid and the possibility for a time of an enhanced supply, as defined in claim 1.

Provided that the reservoir has the desired single opening, it may have any suitable shape and be made from any suitable material. Naturally it must be resistant to the volatile liquid contained therein, i.e., not be chemically degraded, softened or swollen by it. Glass, ceramics, metals and selected plastics may be used, any such selection being within the skill of the art. It is suitably designed so as to define an internal evaporation space. In a typical simple arrangement, the reservoir will have the form of an open-mouthed container, the mouth being closed by the membrane and the quantity of liquid being such that a suitable space is defined. The skilled person will readily be able to envisage many variants of reservoir, both practical and decorative.

The apparatus is configured such that the membrane closing the opening, in continual supply mode, has no direct contact with the liquid in the reservoir, but, when enhanced supply mode is desired, it can be brought into contact therewith. The skilled person can readily configure the apparatus to comply with these requirements. In one simple variant, the opening is located at or near the top of the reservoir, when the device is sitting on a horizontal surface, and the membrane can be brought into contact with the liquid by simply inverting. Naturally, the opening can also be in a side of the vessel. Bringing membrane and liquid into contact may also be achieved by any suitable means, for example, by pivotally mounting the reservoir in a frame.

The membrane may be any membrane that meets the following requirements:
- it must permit liquid and vapour to pass through from the internal evaporation space to the atmosphere;
- it must be of such a constitution that, when brought into contact with the liquid, it can absorb sufficient liquid for evaporation into the atmosphere for the desired time.

The second of these conditions dictates that the membrane must be of a reasonable thickness - very thin membranes of the type currently used in the art will allow the passage of the liquid, but they will not be able to retain sufficient liquid for evaporation for a significant time. However, it cannot be too thick, or volatilised liquid within the reservoir will not pass through. The thickness of the membrane should be in the range of from 0.05-0.4mm.

It also means that the membrane must have a certain permeability. This can be achieved by use of a membrane that is inherently permeable, for example, some polymeric substances that can be made in such a manner. However, preferably the permeability is brought about by the use in the membrane of porous fillers. These will be described more fully hereinunder.

Any material that fulfils the abovementioned requirements may be used in this invention.

Typical suitable materials are described in detail in U.S. Pat. No. 3,351,495. The polyolefin described therein is an ultra-high molecular weight polyolefin, in particular, ultra-high molecular weight polyethylene. It has an average weight-average molecular weight of at least 300,000, preferably at least 1,000,000, and in particular about 4 to 7x10⁶. The standard load melt index of the polyolefin is substantially 0, i.e. it is less than 0.1, and more particularly less than 0.01. The reduced viscosity of the polyolefin is not less than 4.0, and in other embodiments more than 10, and in particular more than 15.

Although polyethylene is the most utilised material, polyolefin mixtures can also be used. In particular, also suitable are polypropylene, polybutene, polystyrene, ethylene/propylene copolymers, ethylene/hexylene copolymers, ethylene/butene copolymers, propylene/butene copolymers, ethylene/propylene/butene copolymers and copolymers of ethylene or propylene with an ethylenically unsaturated monocarboxylic acid, that is to say acrylic acid, methacrylic acid or mixtures thereof.

The membrane material is a microporous, filled polymeric material which is polyolefin. Such materials are commercially available as battery separators. The material according to the invention consists essentially of a homogeneous mixture of 8 to 100 vol. % polyolefin having a molecular weight (weight-average) of at least 300,000, a standard load melt index of substantially 0 and a reduced viscosity of not less than 4.0, 1 to 92 vol. % filler and 1 to 40 vol. % plasticizer.

The membranes mentioned hereinabove have a further substantial advantage when used in connection with fragrances, for example, in air fresheners. Many known membranes are made of hydrophobic materials, such as polyolefins such as polyethylene, polypropylene and blends thereof. Such membranes do not allow a good diffusion of the most polar ingredients of a fragrance. For example, molecules bearing alcohols functions are retained within the container and do not pass properly through the membrane. This is obviously a major constraint for the perfumers, as alcohols such as linaool, phenylethyl alcohol and dihydromyrcenol, and polar solvents, are widely used in such applications.

Filled microporous membranes do not exhibit this selectivity phenomenon, and these are the preferred membranes.

Suitable fillers and plasticizers are known to the art. In this context, reference is again made to U.S. Pat. No. 3,351,495. The particular filler used in the invention is finely-divided silica (silicic acid). The average particle size (diameter) of the filler is the range from 0.01 to about 20 .mu.m, the surface area of the filler being in the range from 30 to 950 m² /g , and particularly at least 100 m² /g. Other fillers that may be used include various mineral fillers, such as clays, zeolites and carbonates, and charcoal.

A high density polyolefin/finely-divided silica membrane is particularly preferred.

The material to be used according to the invention comprises a plasticizer, particularly a water-insoluble oil, in particular process oil.

Particularly desirable ranges of amounts for the homogeneous mixture are 15 to 60, preferably 30 to 45, vol.% polyolefin, and 35 to 80, particularly 50 to 65, vol. % filler, and 1 to 10 vol. % plasticizer.

In addition to the constituents mentioned, the material to be used according to the invention can comprise art-recognised additives, such as antioxidants (usually 0.1 to 1%), lubricants (usually 0.1 to 1%), antistatics, pigments, dyestuffs, conductive carbon black, stabilizers, light stabilizers and the like.

The apparatus used according to the invention comprises an end-of-life indicator. Such an embodiment is very useful, as it can inform when the device needs replacing, or when a charge of liquid on the membrane is exhausted. In this invention, this is achieved by a change in colour of the membrane, that is, a membrane charged with liquid is a different colour from a dry membrane. This can be achieved by any convenient means. For example, it may be achieved by the incorporation in the membrane of a material that interacts with the liquid to produce a colour change. The interacting material must naturally be chosen such that the colour change is reversible.

The ability to change colour depending on the presence or absence of liquid is an inherent property of the membrane, so that it need not be modified to achieve this. This is a property of the preferred polyolefin/silica battery separator-type membranes hereinabove described, and another reason for their particular utility in the working of this invention.

In operation, the apparatus will stand with the membrane out of contact with the liquid. This will ensure a constant supply of liquid to the atmosphere. When an enhanced emission is required, the liquid is brought into contact with the membrane for a time sufficient to charge it with liquid, and the two are then separated. This can be done, for example, by simply inverting the apparatus and then turning it back again. For example, the membrane may be located near a flat top of the apparatus, such that the apparatus can stand upside-down. Alternatively, the apparatus may be mounted in a cradle or on pivots.

The invention is further described with reference to the accompanying drawing. This represents an air freshener containing a fragrance.
Figure 1 is a schematic vertical cross-section through a preferred embodiment.
Figure 2 is a chart, showing the liquid release characteristics of the embodiment over a period of time.

The apparatus, generally indicated as 1 consists of a reservoir 2 that has the shape of a vessel with an opening 3 at one end. The reservoir contains a volatile liquid 4, in this case, a fragrance. The open end of the reservoir is closed with a membrane 5. The membrane is a polyethylene/silica membrane of 0.05-0.4mm thickness (the actual membrane used is Membrane DS2 drying sweat system ex Daramic, Inc). The membrane 5 has a flat top, which allows the apparatus to be inverted and to sit stably on a horizontal surface. Between the membrane 5 and the liquid 4 is an internal evaporation space 6.

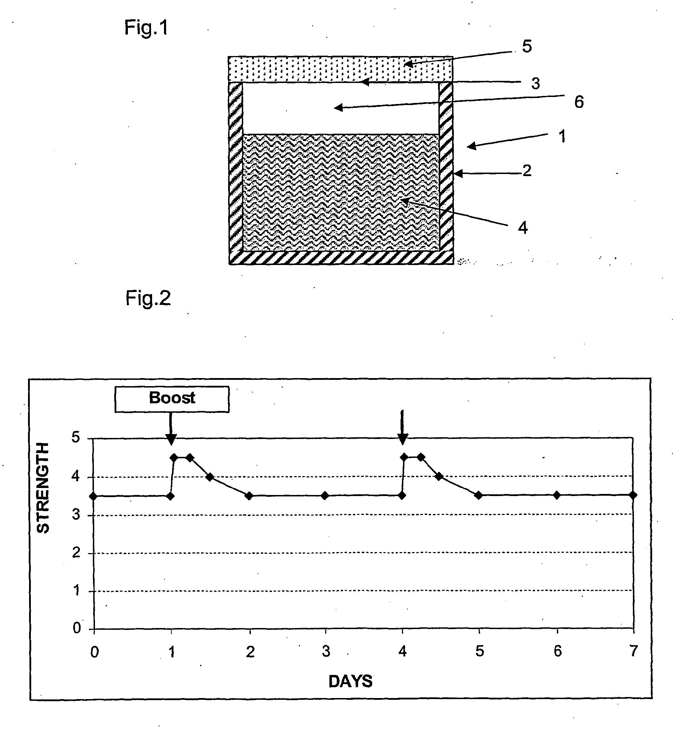

For continual emission of liquid, the apparatus sits as shown in Figure 1, with the membrane out of contact with the fragrance. Fragrance evaporates into the internal evaporation space 6, permeates through the membrane 5 and thus into the atmosphere.

When an enhanced fragrance is presence is desired in the atmosphere, the apparatus is inverted for a time sufficient for it to absorb sufficient liquid, typically for only a second. The membrane changes colour from opaque white to the colour of the liquid and it becomes slightly translucent. The apparatus is set the right way up and volatile liquid commences to emanate from the membrane. The end of life of the particular charge may be observed by observing the colour of the membrane. When it returns to its original colour, it can again be inverted to recharge.

Figure 2 shows graphically the results of measurements taken over a time period of just over a week. The strength figures depicted on the ordinate are assessments of the strength of the fragrance taken at intervals by an experienced panel, as used in the fragrance industry. The strengths are 5 = very strong, 4 = strong, 3 = average, 2 = weak, 1 = very weak, 0 = odourless. At the one-day and four-day marks, the device is inverted to charge the membrane, and the quantity of fragrance rises appreciably. Over a period of about a day, it declines back to the average level.

The skilled person will perceive many possible variations of this invention, which lie within the scope of the invention.

## Claims

1. A method of providing in an atmosphere both a continual supply of volatile liquid and an enhanced supply, comprising providing the liquid (4) in a reservoir (2) with an opening (3), which opening is covered by a membrane (5) of a thickness of from 0.05-0.4mm so as to define within the reservoir an internal evaporation space (6), the continual supply being provided by liquid (4) evaporating within the internal evaporation space (6) and passing through the membrane, and the enhanced supply being provided by evaporation from liquid (4) absorbed in the membrane (5) which has been brought into contact with the liquid and then separated therefrom, the membrane consisting essentially of a homogeneous mixture of 8 to 100 vol. % polyolefin having a molecular weight (weight-average) of at least 300,000, a standard load melt index of less than 0.1 and a reduced viscosity of not less than 4.0, 1 to 92 vol. % filler and 1 to 40 vol. % plasticizer, the filler being finely divided silica and the volatile liquid fragrance comprising alcohol functions.

2. A method according to claim 1, in which the material of the membrane (5) is selected from the group consisting of polyethylene, polypropylene, polybutene, polystyrene, ethylene/propylene copolymers, ethylene/hexylene copolymers, ethylene/butene copolymers, propylene/butene copolymers, ethylene/propylene/butene copolymers.

3. A method according to claim 2, in which the polyolefin is an ultra-high molecular weight polyolefin, preferably an ultra-high molecular weight polyethylene.

4. A method according to any one of claims 1-3, in which the polyolefin has a molecular weight of at least 1,000,000, preferably from 4 - 7x10⁶.

5. A method according to any one of claims 1-4, in which the standard load melt index is less than 0.01, and is preferably effectively 0.

6. A method according to any one of claims 1-5, in which the reduced viscosity of the polyolefin is more than 10, preferably more than 15.

7. A method according to any one of claims 1-6, in which the filler is porous.

## Patentansprüche

1. Verfahren zur Vorsehung sowohl einer fortwährenden Zufuhr von flüchtiger Flüssigkeit als auch einer gesteigerten Zufuhr in einer Atmosphäre, umfassend das Bereitstellen der Flüssigkeit (4) in einem Reservoir (2) mit einer Öffnung (3), wobei die Öffnung von einer Membran (5) mit einer Dicke von 0,05 - 0,4 mm bedeckt ist, um so innerhalb des Reservoirs einen internen Verdampfungsraum (6) zu definieren, wobei die fortwährende Zufuhr durch Flüssigkeit (4) vorgesehen wird, die innerhalb des internen Verdampfungsraums (6) verdampft und durch die Membran hindurchströmt, und die gesteigerte Zufuhr durch Verdampfung von Flüssigkeit (4) vorgesehen wird, die in der Membran (5) absorbiert wird, welche mit der Flüssigkeit in Kontakt gebracht wurde und danach von dieser getrennt wurde, wobei die Membran im Wesentlichen aus einer homogenen Mischung von 8 bis 100 Vol.-% Polyolefin mit einem Molekulargewicht (Gewichtsmittel) von mindestens 300 000, einem standardmäßigen Ladungsschmelzindex von weniger als 0,1 und einer reduzierten Viskosität von nicht weniger als 4,0, 1 bis 92 Vol.-% Füllstoff und 1 bis 40 Vol.-% Weichmacher besteht, wobei der Füllstoff fein zerteiltes Silica ist und der flüchtige flüssige Duftstoff Alkoholfunktionen umfasst.

2. Verfahren gemäß Anspruch 1, in dem das Material der Membran (5) aus der Gruppe gewählt ist, die aus Polyethylen, Polypropylen, Polybuten, Polystyrol, Ethylen/Propylen-Copolymeren, Ethylen/Hexylen-Copolymeren, Ethylen/Buten-Copolymeren, Propylen/Buten-Copolymeren und Ethylen/Propylen/Buten-Copolymeren besteht.

3. Verfahren gemäß Anspruch 2, in dem das Polyolefin ein ultrahochmolekulargewichtiges Polyolefin, vorzugsweise ein ultrahochmolekulargewichtiges Polyethylen ist.

4. Verfahren gemäß einem beliebigen der Ansprüche 1 - 3, in dem das Polyolefin ein Molekulargewicht von mindestens 1 000 000, vorzugsweise von 4 - 7 x 10⁶, besitzt.

5. Verfahren gemäß einem beliebigen der Ansprüche 1 - 4, in dem der standardmäßige Ladungsschmelzindex weniger als 0,01 beträgt und vorzugsweise effektiv 0 ist.

6. Verfahren gemäß einem beliebigen der Ansprüche 1 - 5, in dem die reduzierte Viskosität des Polyolefins mehr als 10, vorzugsweise mehr als 15 beträgt.

7. Verfahren gemäß einem beliebigen der Ansprüche 1 - 6, in dem der Füllstoff porös ist.

## Revendications

1. Procédé pour fournir dans une atmosphère à la fois un apport continu de liquide volatil et un apport accru, comprenant la disposition du liquide (4) dans un réservoir (2) muni d'un orifice (3), lequel orifice est couvert avec une membrane (5) ayant une épaisseur de 0,05 à 0,4 mm de manière à définir dans le réservoir un espace d'évaporation interne (6), l'apport continu étant produit par le liquide (4) s'évaporant dans l'espace d'évaporation interne (6) et traversant la membrane, et l'apport accru étant produit par évaporation à partir du liquide (4) absorbé dans la membrane (5) qui a été mise en contact avec le liquide et ensuite séparée de celui-ci, la membrane consistant essentiellement en un mélange homogène de 8 à 100 % en volume d'une polyoléfine ayant une masse moléculaire (moyenne en poids) d'au moins 300 000, un indice de fluidité à chaud sous charge normalisée inférieur à 0,1 et une viscosité réduite non inférieure à 4,0, 1 à 92 % en volume de charge et 1 à 40 % en volume de plastifiant, la charge consistant en silice finement divisée et le parfum liquide volatil comprenant des fonctions alcool.

2. Procédé selon la revendication 1, dans lequel le matériau de la membrane (5) est choisi dans le groupe constitué par le polyéthylène, le polypropylène, le polybutène, le polystyrène, des copolymères éthylène/propylène, des copolymères éthylène/hexylène, des copolymères éthylène/butène, des copolymères propylène/butène, des copolymères éthylène/propylène/butène.

3. Procédé selon la revendication 2, dans lequel la polyoléfine est une polyoléfine à ultra-haute masse moléculaire, de préférence un polyéthylène à ultra-haute masse moléculaire.

4. Procédé selon l'une quelconque des revendications 1 à 3, dans lequel la polyoléfine a une masse moléculaire d'au moins 1 000 000, de préférence de 4 - 7 x 10⁶_{.}

5. Procédé selon l'une quelconque des revendications 1 à 4, dans lequel l'indice de fluidité à chaud sous charge normalisée est inférieur à 0,01, et est de préférence en réalité 0.

6. Procédé selon l'une quelconque des revendications 1 à 5, dans lequel la viscosité réduite de la polyoléfine est supérieure à 10, de préférence supérieure à 15.

7. Procédé selon l'une quelconque des revendications 1 à 6, dans lequel la charge est poreuse.
